# EUROPEAN PATENT APPLICATION

(11) **EP 1 522 262 A1**
(43) Date of publication of application: **13.04.2005**
(21) Application number: 04024006.1
(22) Date of filing: 08.10.2004
(51) Int. Cl.: A61B 17/06

(54) **High strength suture kit for shoulder fracture repair**

(30) Priority: 10.10.2003 US 509950 P
(71) Applicant: Arthrex, Inc., Naples, Florida 34108-1945 (US)
(72) Inventor: Romeo, Anthony A., Willowbrook IL 60527 (US)
(74) Representative: Crippa, Paolo Ernesto

(57) **Abstract**

High strength suture kits and products related to shoulder fracture repair using a shoulder prosthesis. An exemplary kit contains color-coded lengths of high-strength suture and a copy of a complementary surgical plan represented and detailed by a written brochure, and an audio and/or video recording, for example. The complementary surgical plan involves pre-installing the color-coded suture, which is used for horizontal cerclage, temporary sutures, and vertical tension bands. The fractured humeral head is replaced with the prosthetic humerus, which is secured by the shoulder tuberosities using the pre-installed suture.

## Description

### BACKGROUND OF THE INVENTION

The present invention relates to shoulder fracture repair, and more particularly to prosthetic replacement of a fractured humerus using a high strength suture kit.

Procedures for shoulder reconstructions using a shoulder prosthesis are known. Techniques are disclosed in Boileau et al., Tuberosity Osteosynthesis and Hemiarthroplasty for Four-Part Fractures of the Proximal Humerus. It would be advantageous to improve upon the Boileau et al. technique, and to provide a kit including color-coded, high strength sutures for carrying out the reconstruction.

### BRIEF SUMMARY OF THE INVENTION

The present invention provides apparatus and methods for shoulder reconstruction using a shoulder prosthesis. The apparatus includes a kit of color-coded lengths of high-strength suture and a complementary surgical plan for carrying out the reconstruction. The method includes pre-installing color-coded suture to be used for horizontal cerclage and vertical tension, replacing the fractured humeral head with a prosthetic humerus, and securing the pre-installed suture to fix the shoulder tuberosities around the prosthesis.

### BRIEF DESCRIPTION OF THE DRAWINGS

Fig. 1 illustrates the step of pre-installing cerclage sutures in connection with a shoulder arthroplasty in accordance with the present invention;

Fig. 2 illustrates the step of installing temporary sutures and tensions sutures in connection with a shoulder arthroplasty in accordance with the present invention;

Fig. 3 illustrates the step of removing a fractured humeral head in connection with a shoulder arthroplasty in accordance with the present invention;

Fig. 4 illustrates the step of installing a prosthetic humeral head in connection with a shoulder arthroplasty in accordance with the present invention;

Fig. 5 illustrates the step of tensioning cerclage suture in connection with a shoulder arthroplasty in accordance with the present invention;

Fig. 6 illustrates the step of securing cerclage suture in connection with a shoulder arthroplasty in accordance with the present invention;

Fig. 7 illustrates the step of threading cerclage around the lesser tuberosity in connection with a shoulder arthroplasty in accordance with the present invention;

Fig. 8 illustrates the step of packing the prosthetic humeral neck with cancellous bone in connection with a shoulder arthroplasty in accordance with the present invention;

Fig. 9 illustrates the step of securing additional cerclage suture in connection with a shoulder arthroplasty in accordance with the present invention;

Fig.10 illustrates the step of interval closure in connection with a shoulder arthroplasty in accordance with the present invention;

Fig. 11 illustrates the step of securing vertical tension sutures in connection with a shoulder arthroplasty in accordance with the present invention; and

Fig. 12 illustrates a kit including high-strength suture and a complementary surgical plan for performing the surgical method according to the present invention.

### DETAILED DESCRIPTION OF THE INVENTION

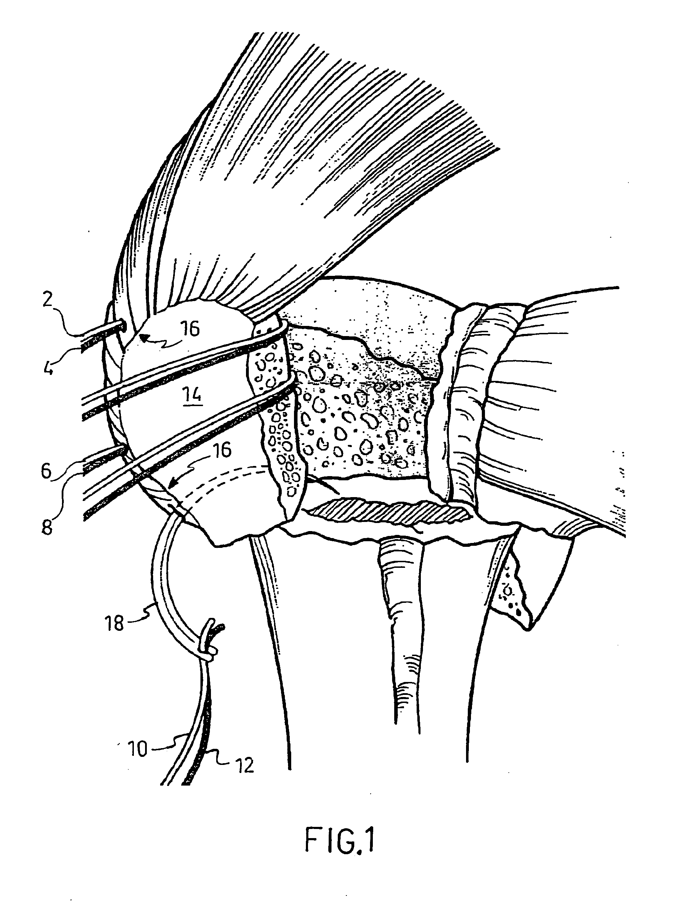

Referring initially to Fig. 1, with the arm in abduction/internal rotation, three pairs 2:4, 6:8, 10:12 of high strength sutures are inserted around the greater tuberosity 14 through the bone-infraspinatus tendon junction 16 using a needle 18. Pairs of sutures 2:4,6:8,10:12 are coded to distinguish the sutures in each pair. In an exemplary embodiment, sutures 2, 6, 10 are white, and sutures 4, 8, 12 are blue. The preferred high strength suture is FiberWire suture, sold by Arthrex, Inc. of Naples, Florida, and described in U.S. Patent U.S. Patent No. 6,716,234, issued April 6, 2004 to Grafton et al. as a high strength surgical suture material with improved tie down characteristics. The suture features a braided cover made of a blend of ultrahigh molecular weight long chain polyethylene and polyester. The polyethylene provides strength. The polyester provides improved tie down properties.

An exemplary suture strand includes a multifilament cover formed of a plurality of fibers of ultrahigh molecular weight polyethylene braided with fibers of polyester. The cover surrounds a core of twisted fibers of ultrahigh molecular weight polyethylene. Preferably, the ultrahigh molecular weight polyethylene includes about 60% of the cover fibers, with polyester making up about 40% of the cover filaments. The core comprises about 30% of the suture, the cover making up about 70%. As an enhancement, the suture is provided with a coating on the cover, as is known in the prior art. Ultrahigh molecular weight polyethylene fibers suitable for use in the suture are marketed under the Dyneema trademark by Toyo Boseki Kabushiki Kaisha.

The suture advantageously has the strength of Ethibond #5 suture, yet has the diameter, feel and tie ability of #2 suture. As a result, the suture is ideal for most orthopedic procedures such as rotator cuff repair, Achilles tendon repair, patellar tendon repair, ACL/PCL reconstruction, hip and shoulder reconstruction procedures, and replacement for suture in anchors. FiberWire suture includes a multifilament cover featuring braided strands of ultrahigh molecular weight long chain polyethylene and polyester, and surrounding a core of twisted fibers of ultrahigh molecular weight polyethylene. Different sutures can be distinguished from one another by using various colors and patterns of fibers and strands in forming the suture construct.

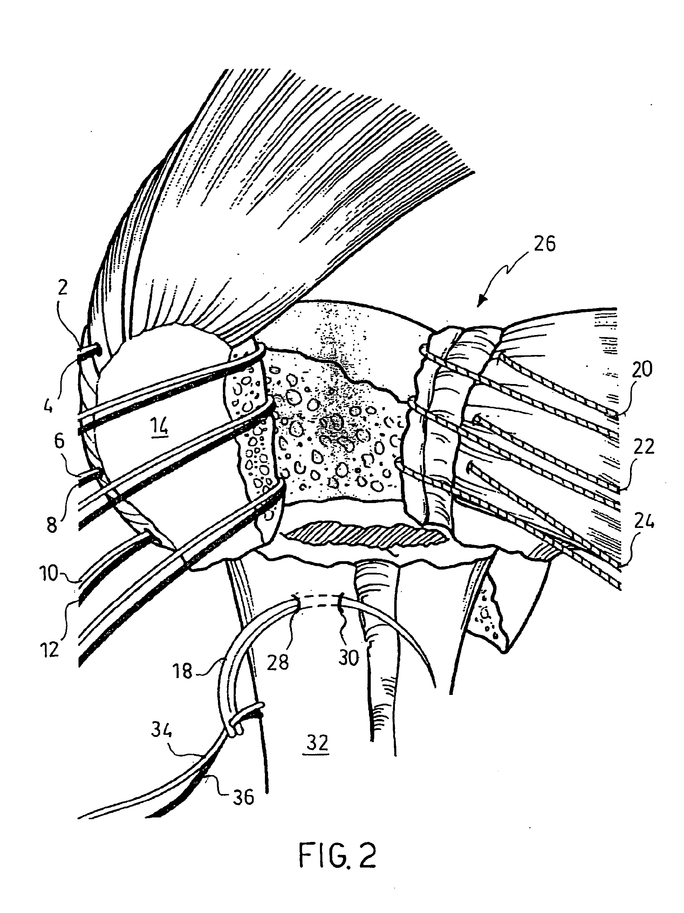

Referring to Fig. 2, three temporary sutures 20, 22, 24 are inserted through the lesser tuberosity 26, again at the bone-tendon junction. Temporary sutures 20, 22, 24 are striped, for example, to distinguish them from the blue and white pairs of suture inserted around the greater tuberosity. Drill holes 28,30, are formed in the proximal shaft of the humerus 32 to a diameter of 2.5 mm. Another pair of bi-colored high-strength sutures 34, 36 is inserted through holes 28, 30 using needle 18. Sutures 34, 36 will be used as vertical tension sutures.

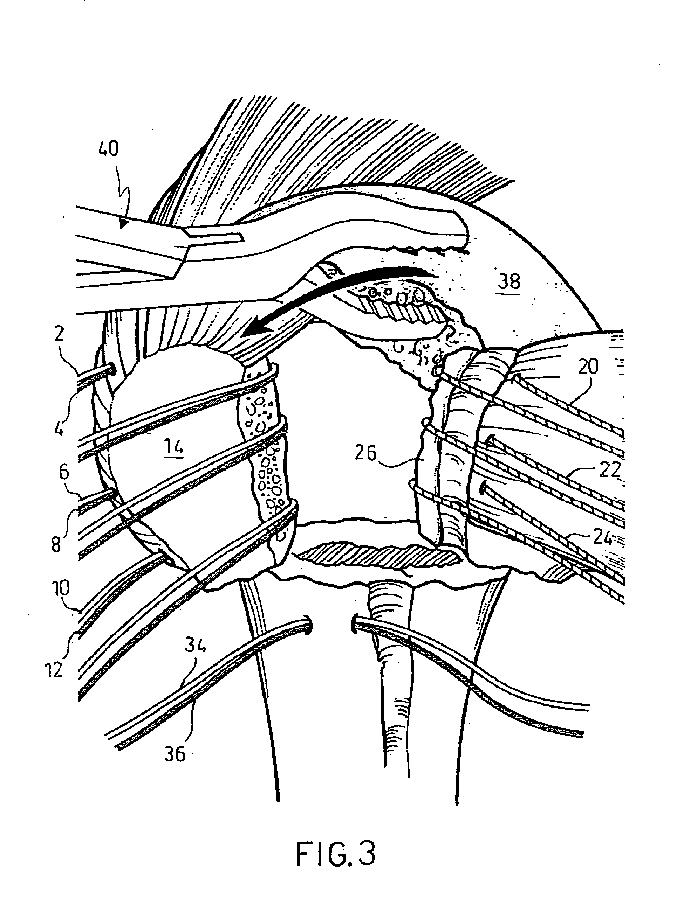

Referring to Fig. 3, the greater and lesser tuberosities 14, 26 are gently retracted and the humeral head fragment 38 is removed using forceps 40 after resection of any capsular attachments. The glenoid and other structures of the joint are inspected, and indicated repairs are undertaken to avoid recurrent postoperative instability.

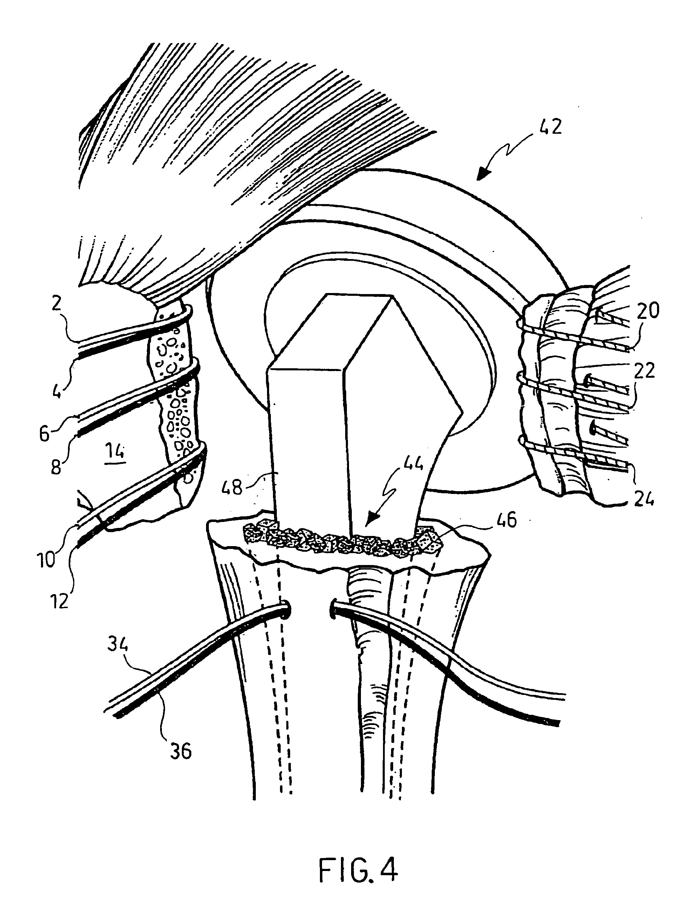

Referring to Fig. 4, an appropriately sized prosthetic humeral head 42 is chosen and reduced into the joint after appropriate measurement of the humeral head 38 and preparation of the medullary canal 44. Positioning of the prosthesis can be achieved by the use of appropriate instrumentation (not shown). The prosthesis preferably is cemented in place, and cancellous bone graft 46 harvested from the humeral head 38 is packed into the medullary space around the prosthetic neck 48 to facilitate bony union.

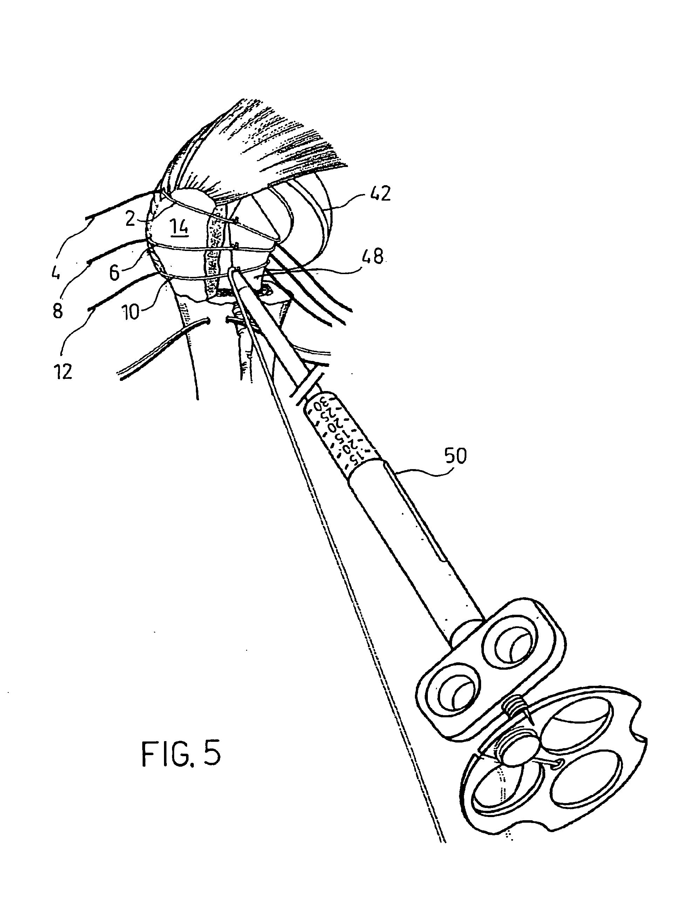

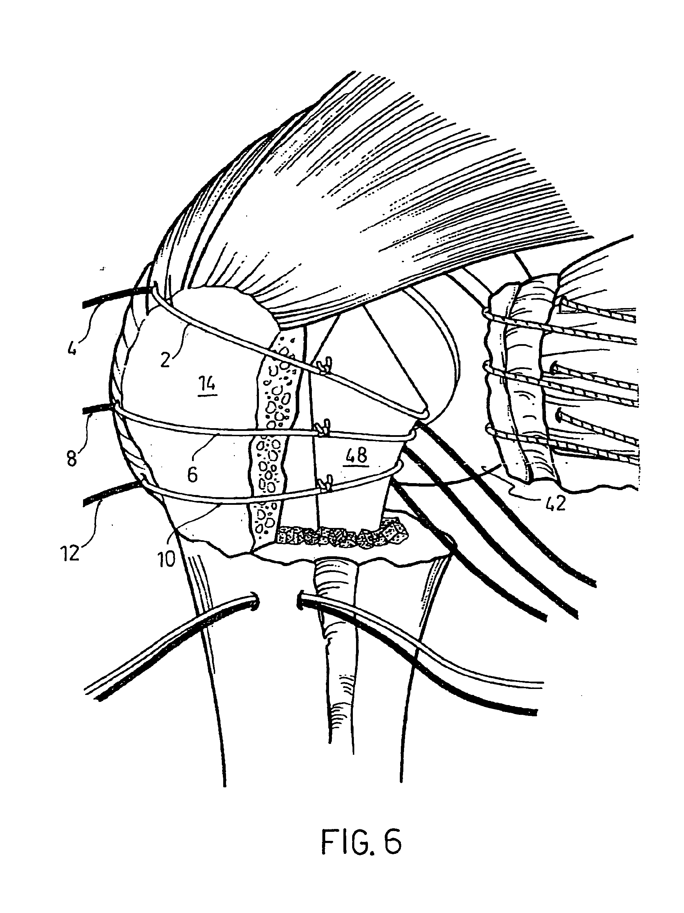

Referring to Figs. 5 and 6, reattachment of greater tuberosity 14 begins by tying white sutures 2, 6, 10 horizontally around prosthetic neck 48. A suture tensioning device 50, shown in Fig. 5, is utilized to apply the appropriate tension to each length of cerclage suture.

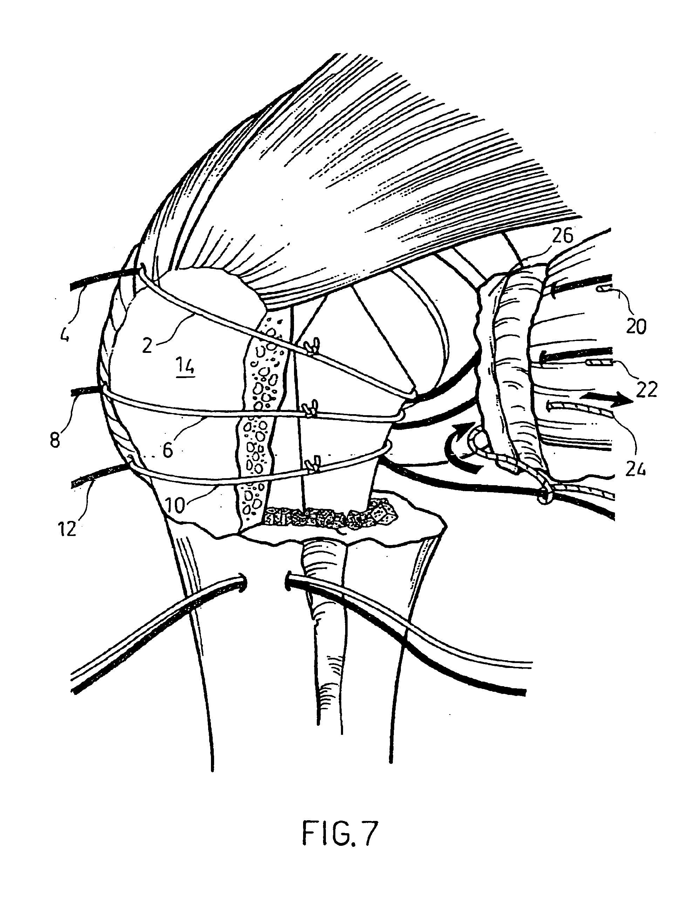

Referring to Fig. 7, reattachment of the lesser tuberosity begins by drawing ends of each of the blue sutures 4, 8, 12 using temporary striped sutures 20, 22, 24 through holes formed in tissue of the lesser tuberosity 26. The directional arrows in Fig. 7 illustrate how blue suture 12 is attached to temporary striped suture 24 to be drawn through the subscapularis tendon from inside to outside.

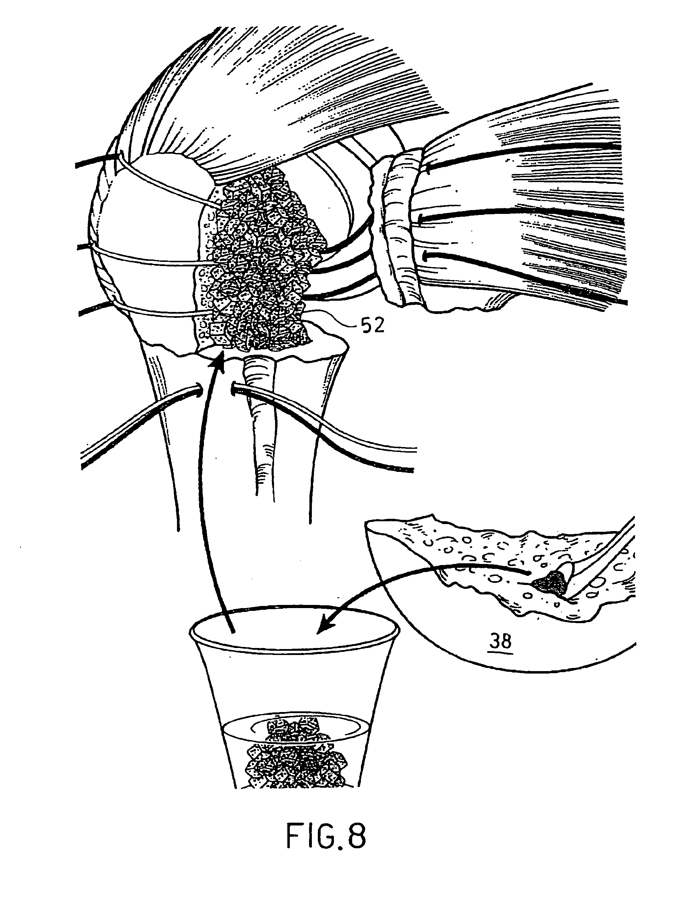

Referring to Fig. 8, prior to pulling the lesser tuberosity into position under the prosthetic head, cancellous bone 52 harvested from the humeral head 38 is packed around the prosthetic neck 48.

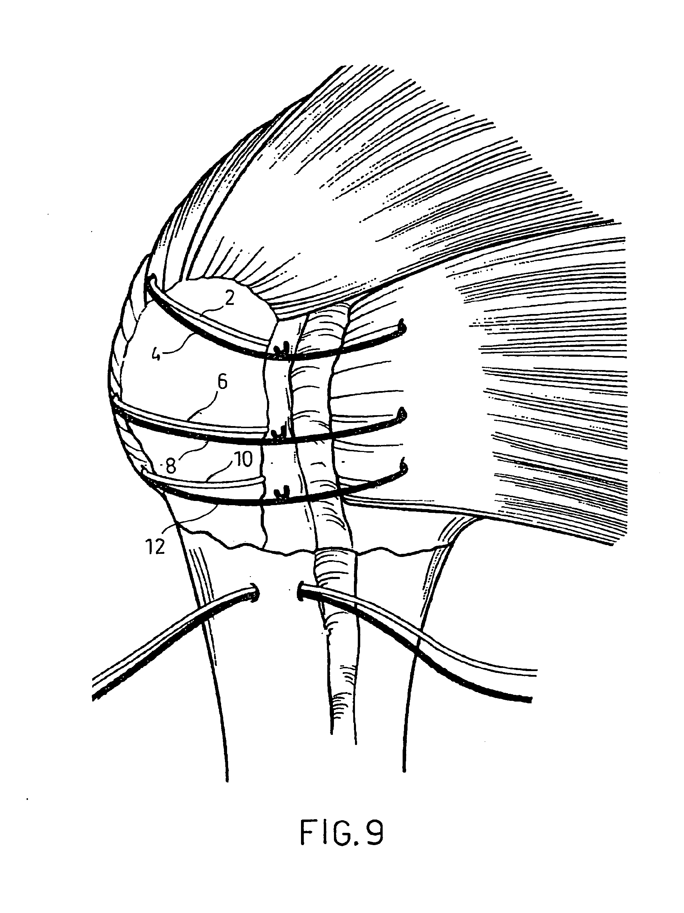

Referring to Fig. 9, lesser tuberosity 26 is pulled into place using horizontal cerclage sutures 4, 8, 12.

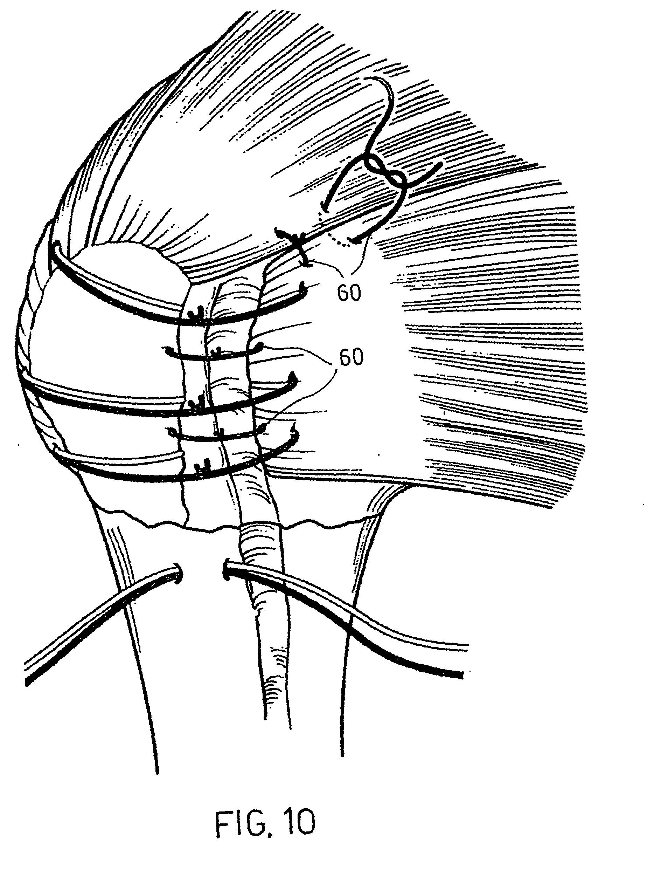

Referring to Fig. 10, soft tissue and rotator cuff intervals are dosed using lengths 60 of high-strength suture (#2 FiberWire) with needles.

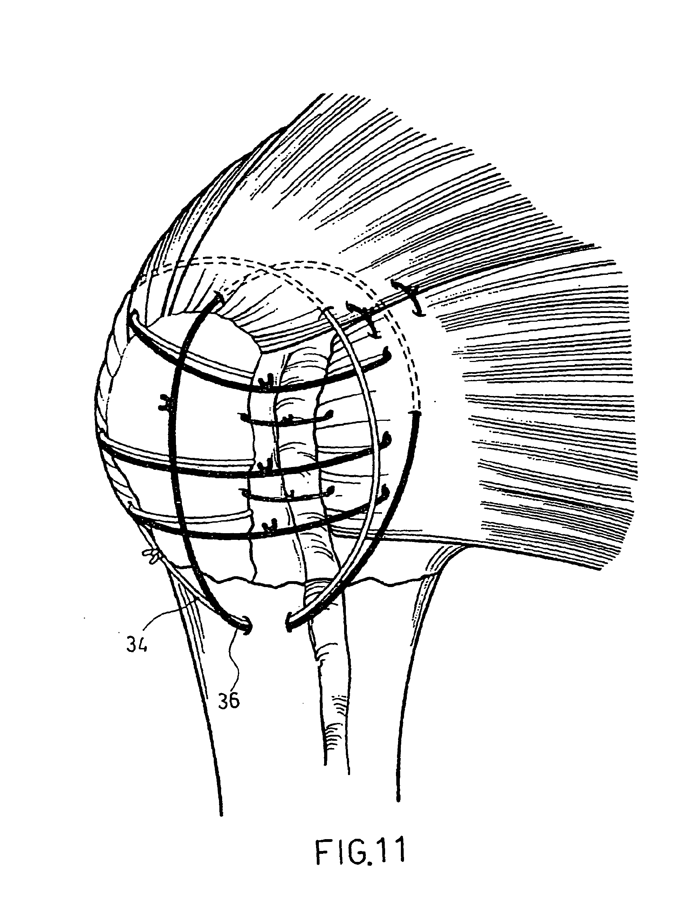

Referring to Fig. 11, sutures 34, 36 are used as vertical tension bands. One suture is passed anteriorly through the subscapularis and the supraspinatus tendons. The other suture is passed posteriorly through the infraspinatus and supraspinatus tendons. The vertical tension bands provide important fixation of the tuberosity fragments to the humeral shaft

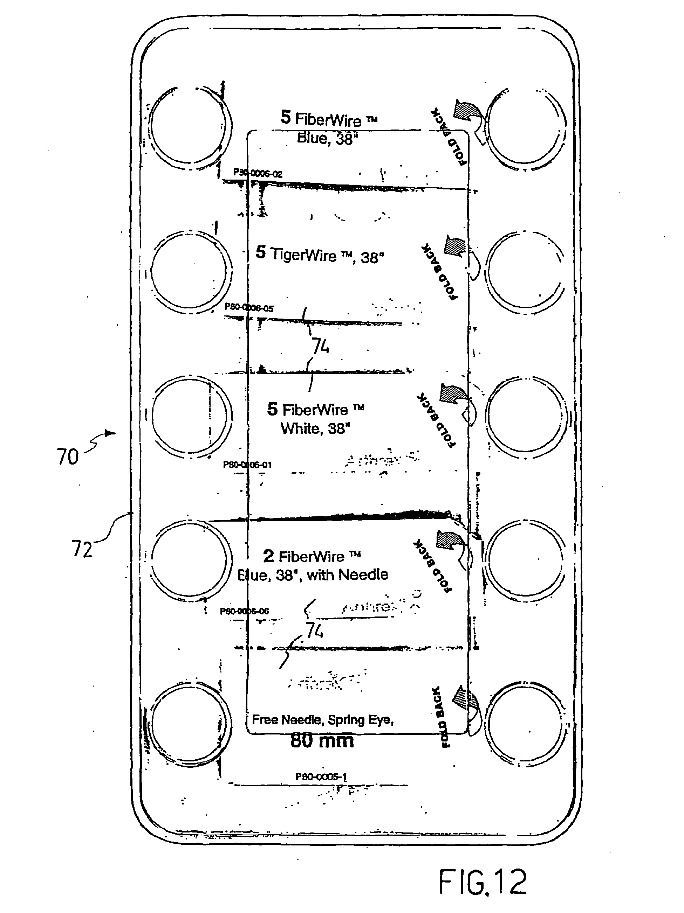

Referring to Fig. 12, a high-strength suture kit 70 is illustrated. Suture kit 70 includes a tray 72 containing individual packages 74 of color-coded high-strength sutures pre-cut for use as cerclage, temporary suture, and vertical tension bands, needles, and combinations thereof sufficient to carry out the shoulder reconstruction discussed above. Also included in kit 70 is a surgical plan outlining the steps of performing the reconstruction. In an exemplary embodiment, the surgical plan is a printed brochure featuring graphical representations and written instructions such as those depicted in Figs. 1-11 and described above. Alternatively, or in addition thereto, the surgical plan can be provided as an audio and/or video recording, for example. The surgical plan recording can be provided for playback on a removable disc, such as a CD or DVD, for example, or the kit can provide means and/or instructions for accessing the recording over a network, such as the Internet.

Although the present invention has been described in connection with preferred embodiments, many modifications and variations will become apparent to those skilled in the art.

## Claims

1. A kit for shoulder reconstruction comprising:
lengths of high-strength suture pre-cut for use as shoulder horizontal cerclage, temporary sutures, and vertical tension bands;and
a complementary surgical plan for carrying out the shoulder reconstruction.

2. A kit according to claim 1, wherein the high-strength suture is color coded, and the complementary surgical plan comprises at least one of written, audio, and video instructions for pre-installing suture to be used for horizontal cerclage and vertical tension, replacing a fractured humeral head with a prosthetic humerus, and securing the pre-installed suture to secure the shoulder tuberosities around the prosthesis.

3. A kit according to claim 1, wherein the high strength suture includes multifilament strands of ultrahigh molecular weight long chain polyethylene and polyester fibers.

4. A kit according to claim 3, wherein the high strength suture includes a multifilament cover featuring braided strands of ultrahigh molecular weight long chain polyethylene and polyester, over a core of twisted fibers of ultrahigh molecular weight polyethylene.

5. A kit according to claim 1, further comprising a prosthetic joint portion.

6. A kit according to claim 1, further comprising a prosthetic humeral head.

7. A reconstructed shoulder comprising:
pre-installed suture for horizontal cerclage and vertical tension, a fractured humeral head having been replaced by a prosthetic humerus; and shoulder tuberosities secured around the prosthesis with the pre-installed suture.

8. A reconstructed shoulder according to claim 7, wherein the suture is color-coded.

9. A reconstructed shoulder according to claim 7, comprising a set of suture installed to the greater tuberosity and another set of suture installed to the lesser tuberosity.

10. A reconstructed shoulder according to claim 9, further comprising vertical tension bands of lengths of highstrength suture.
